# EUROPÄISCHE PATENTANMELDUNG

(11) **EP 1 892 000 A1**
(43) Veröffentlichungstag der Anmeldung: **27.02.2008**
(21) Anmeldenummer: 06119308.2
(22) Anmeldetag: 22.08.2006
(51) Int. Cl.: A61M 1/36, A61M 1/16, A61M 1/34

(54) **Verfahren zum Vorbereiten des Dialysators eines Dialysegerätes**

(71) Anmelder: B. Braun Medizintechnologie GmbH, 34212 Melsungen (DE)
(72) Erfinder: Moll, Stefan, 34212, Melsungen (DE); Roessler, Rudolf, 34286, Spangenberg (DE)
(74) Vertreter: Selting, Günther

(57) **Zusammenfassung**

Zum Vorbereiten enes Filters, z.B. Dialysators, wird elne Spülflüssigkeit, die einem Reservoir (29) entnommen wird, durch eine Pumpe (22) dem Filter (10) zugeführt. In einer Füllphase wird die Spülflüssigkeit mit niedriger Fließrate zugeführt, um den Filter (10) zu entlüften. In einer nachfolgenden Spülphase, in der Fremdkörper aus dem Filter ausgespült werden, wird die Fließrate erhöht. Auf diese Weise erfolgt eine Entkopplung der Spülphase von der langsameren Füllphase und somit insgesamt eine Verkürzung der Vorbereitungszeit.

## Beschreibung

Die Erfindung betrifft ein Verfahren zum Vorbereiten des Dialysators eines Dialysegerätes, das einen Blutkreislauf und einen Dialysierflüssigkeitskreislauf aufweist, wobei der Dialysator eine Blutkammer und eine Dialysierflüssigkeitskammer enthält, zwischen denen sich eine semipermeable Membran befindet. Generell betrifft die Erfindung Therapiesysteme zur Nierenersatz-Therapie.

Dialysatoren werden in trockenem Zustand geliefert. Die Membran des Dialysators ist somit beim Aufrüsten des Gerätes ebenfalls trocken. Im Vorbereiten der Therapie wird der Dialysator im Zusammenspiel mit dem Dialysegerät zunächst mit einer wässrigen physiologischen Lösung gefüllt und durchflossen. Dieser Vorgang wird als *Dialysator-Vorbereitung* (priming) bezeichnet. Das Vorbereiten des Dialysators bewirkt zum einen, dass die trockene Membran so mit Flüssigkeit gefüllt wird, dass keine Luft an oder in der Membran zurück bleibt. Luft im Dialysator reduziert die Effizienz der Membran und damit der Therapie. Zum anderen sollen durch den gleichzeitigen oder nachfolgenden Spülvorgang Reststoffe aus der Membran herausgespült werden. Diese Reststoffe können dem Produktions-, Verpackungs- oder Aufrüstprozess entstammen und stellen eine potentielle Gefährdung des Patienten dar. Nachdem der Dialysator und mit ihm das Schlauchsystem vorbereitet ist, wird der Patient angelegt und die wässrige Flüssigkeit wird aus dem vollständig gefüllten System abgelassen und auf einer Seite durch das Blut des Patienten ersetzt.

Das Füllverhalten verschiedener Dialysatoren unterscheidet sich stark. Häufig müssen verbleibende Luftblasen durch Klopfen aus dem Dialysator entfernt werden. Je nach Produktionsverfahren sind unterschiedliche Spülmengen nötig. Daher arbeiten heutige Dialysegeräte mit einem Fluss beim Vorbereiten des Dialysators von 100 ml/min und einer Gesamtmenge von rund 1.000 ml. Der relativ geringe Fluss sorgt für eine gute Entlüftung des Dialysators, auch bei Dialysatoren mit schlechtem Entlüftungsverhalten. Die Gesamt-Flüssigkeitsmenge von 1.000 ml stellt auch bei Dialysatoren mit hohem Bedarf an spülflüssigkeit eine Entfernung aller Reststoffe sicher. Die Parameter zum Vorbereiten des Dialysators sind in der Regel fest eingestellt und können vom Anwender nicht oder nur schwierig verändert werden.

In WO 02/098491 A1 (Gambro Lundia) ist ein Verfahren zum Füllen und Spülen eines Filters einer Dialysemaschine beschrieben. Dabei erfolgt eine Rezirkulation einer physiologischen Spülflüssigkeit in dem Dialysatkreislauf und eine Ubertragung der Spülflüssigkeit durch die Membran des Filters in den Blutkreislauf. Die Vorbereitungsphase ist von einer Steuereinheit gesteuert, die Pumpen in dem Dialysatkreislauf derart betreibt, dass zunächst der Filter gefüllt wird. Anschließend erfolgt in einer zweiten Phase eine Spülung der Membran. Das Verfahren ist auf eine Reduzierung der Spülflüssigkeitsmenge durch transmembranes Spülen gerichtet. Die heute benutzten Dialysatoren erlauben jedoch keine großen Transmembranflüsse, so dass dieser Vorgang zeitaufwändig ist.

EP 0 831 945 B1 (Gambro) beschreibt das Vorbereiten eines Dialysators mit einer sterilen Salzlösung zum Entfernen von Luft aus dem extrakorporalen Kreislauf. Zusätzlich erfolgt das Spülen des Dialysators, wobei die Spülflüssigkeit für eine bestimmte Zeit durch den Dialysator rezirkuliert wird. Während der Entlüftung und während des Spülens erfolgt die Förderung der Spülflüssigkeit durch die im Blutkreislauf enthaltene Blutpumpe.

EP 1 323 439 A2 (Medical Support) beschreibt ein Vorbereitungsverfahren zum Befüllen oder Freispülen eines extrakorporalen Blutkreislaufs von Dialysemaschinen, bei dem ein maschinenspezifisches Füllprogramm abläuft. Im Rahmen des Steuerprogramms wird Dialysierflüssigkeit durch die Membran des Dialysators hindurchgefördert. Dies stellt ebenfalls eine transmembrane Spülung dar.

Der Erfindung liegt die Aufgabe zugrunde, ein Verfahren zum Vorbereiten des Dialysators eines Dialysegerätes anzugeben, bei dem die für die Vorbereitung insgesamt benötigte Zeit verkürzt ist.

Das erfindungsgemäße Verfahren ist durch den Patentanspruch 1 definiert. Es weist die folgenden Schritte auf:
a) Füllen der Blutkammer des Filters mit einer Spülflüssigkeit mit einer ersten Fließrate, die so niedrig ist, dass Luftblasen sich ablösen und entweichen können, und
b) Spülen des Filters durch Hindurchleiten der Spülflüssigkeit durch die Blutkammer mit einer zweiten Fließrate, die größer ist als die erste Fließrate.

Die Erfindung beruht auf dem Gedanken, dass zur Reduzierung der Vorbereitungszeit des Dialysators eine Entkopplung der Füll- und Entlüftungsphase und der Spülphase erfolgt, wobei in beiden Phasen mit unterschiedlichen Flüssen gearbeitet wird. Hierbei kann die Füllphase mit der benötigten Langsamkeit durchgeführt werden, ohne dass hierdurch die Dauer der Spülphase verlängert wird.

Die Filter oder Dialysatoren bestehen aus einem Hahlfaserbündel mit Kapillaren, deren Durchmesser etwa 200 µm beträgt. Die Kapillaren werden innen von Blut durchflossen, während der Außenraum die Dialysierflüssigkeitskammer bildet. Von besonderer Wichtigkeit ist die vollständige Entlüftung der Blutkammer, die aus den Innenräumen der Kapillaren besteht. Um hier eine Entlüftung sicherzustellen, darf das Befüllen nicht zu schnell erfolgen, weil sonst Lufteinschlüsse verbleiben könnten. Beim Spülen geht es hingegen darum, eine bestimmte Spülflüssigkeitsmenge durch den Blutkreislauf zu treiben, um dort vorhandene Partikel mitzunehmen. Daher kann das Spülen mit einer erheblich größeren Fließrate erfolgen als die Entlüftung. Die Entlüftungsphase ist nach dem vollständigen Befüllen des Filtergehäuses bzw. der Blutkammer beendet.

Bei einem realistischen Beispiel trägt während der Füllphase der Fluss 100 ml/min. Bei einem blutseitigen Füllvolumen von etwa 100 ml des Gehäuses kann die Entlüftungsphase nach 1:12 Minuten abgeschlossen sein. Die anschließende Spülphase erfolgt mit deutlich höherer Fließrate von hier 400 ml/min. Eine Spülphase mit einer Spülmenge von 880 ml ist somit nach 132 Sekunden beendet. Dies führt zu einer Reduzierung der Zeitdauer des Vorbereitens von 10 min (bei 100 ml/min Fließrate und 1.000 ml Spülmenge) auf 3:24 min bei gestufter Fließrate.

Der Übergang zwischen der Phase des Füllens und der Phase des Spülens kann zeitgesteuert oder in Abhängigkeit von der Menge der geförderten Spülflüssigkeit erfolgen.

Eine Pulsation der Fließrate oder das Erzeugen von Druckimpulsen während der Füll- und Entlüftungsphase des Filters ermöglichen eine höhere Fließrate bei der Entlüftung und damit eine weitere Reduzierung der Zeit. Auch während der Spülphase der Filtervorbereitung können Druckstöße und ein dynamisches Verändern der Fließrate zu einer schnelleren Entfernung der Reststoffe und Verunreinigungen führen. Auch dies bewirkt eine Reduzierung der benötigten Zeit.

Die Pulsation der Fließrate erfolgt durch Starten und Stoppen der Blut- oder Flusspumpe. Eine Pulsation kann aber auch durch einen Druckaufbau erfolgen, der z.B. durch Öffnen und Schließen eines Ventils, beispielsweise der venösen Klemme, erfolgt. Durch Öffnen des Ventils wird dann der abgebaute Druck entlassen.

Alle genannten Parameter, wie die Dauer der Füllphase, die Fließrate der Füllphase, die Dauer der spülphase, die Fließrate während der Spülphase, die Spülmenge und die Pulsationsart sind abhängig vom Typ des verwendeten Filters, dessen Membran-Beschaffenheit und der Größe und Form des Filtergehäuses. Es wäre daher sinnvoll, an der Steuereinheit des Dialysegerätes diese Parameter manuell eingeben zu können oder den Filtertyp einzugeben, dessen Parameter in dem Dialysegerät gespeichert sind. Bei der Auswahl des entsprechenden Filtertyps wird das optimale Parameter-Set zur Filtervorbereitung übernommen. Die Auswahl des entsprechenden Filters kann über eine Liste, eine Eingabe oder über eine direkte Übermittlung vom Filter mit Radiotags oder eine maschinenlesbare Beschriftung erfolgen. Ferner ist die Übernahme des Dialysatortyps oder auch des Parameter-Sets zum Vorbereiten des Filters von einem externen Speichermedium, wie beispielsweise einer Diskette oder Chipkarte, oder über eine Netzwerkverbindung möglich.

Die Erfindung betrifft ferner ein Dialysegerät mit einer Steuereinheit, die den Ablauf des erfindungsgemäßen Verfahrens automatisch steuert.

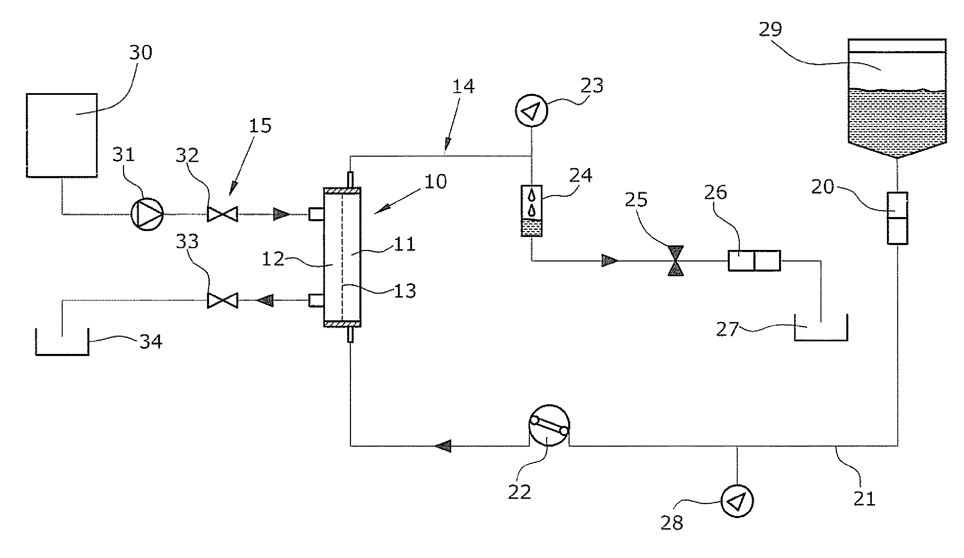

Im Folgenden wird unter Bezugnahme auf die einzige Figur der Zeichnung ein Ausführungsbeispiel näher erläutert,

In der Zeichnung ist ein Diagramm eines Dialysegerätes schematisch dargestellt,

Das Dialysegerät weist einen Filter 10 auf, bei dem es sich vorzugsweise um einen Dialysator handelt. Der Filter 10 kann aber auch aus einer anderweitigen Filtervorrichtung bestehen. Der Filter 10 ist hier schematisch mit einer Blutkammer 11 und einer Dialysierflüssigkeitskammer 12 dargestellt, die durch eine Membran 13 voneinander getrennt sind. In der Praxis besteht der Filter 10 aus einem Hohlfaserbündel mit zahlreichen parallelen Hohlfasern, deren Innenräume die Blutkammer bilden, und deren Außenraum die Dialysierflüssigkeitskammer 12 bilden.

Die Blutkammer 11 ist Bestandteil eines extrakorporalen Blutkreislaufs 14. Die Dialysierflüssigkeitskammer 12 ist Bestandteil eines Dialysierflüssigkeitskreislaufs 15.

Der Blutkreislauf weist eine arterielle Anschlussvorrichtung 20 auf, die über einen Schlauch 21 mit dem unteren Ende der Blutkammer 11 verbunden ist. An den Schlauch 21 ist ein arterieller Drucksensor 28 angeschlossen. Im Verlauf des Schlauches 21 befindet sich eine volumetrische Pumpe 22, bei der es sich um eine übliche Blutpumpe in Form einer Schlauchpumpe handelt. Das obere Ende der Blutkammer 11 ist mit einem Drucksensor 23 und einer Tropfkammer 24 verbunden. Hinter der Tropfkammer 24 befindet sich eine venöse Absperrklemme 25, Eine venöse Anschlussvorrichtung 26 dient zum Anschluss einer venösen Nadel zum Zurückführen des Blutes zum Patienten.

In der Vorbereitungsphase sind die Anschlussvorrichtungen 20 und 26 nicht mit dem Patientenkörper verbunden. Die Anschlussvorrichtung 20 ist mit einem Spülflüssigkeitsreservoir 29 verbunden und die Anschlussvorrichtung 26 ist mit einem Sumpf 27 oder beispielsweise einem Auffangbeutel oder einem Ablauf verbunden. Das Spülflüssigkeitsreservoir kann auch über die Maschine im Online Verfahren zur Verfügung gestellt werden. Auch auf diesem Weg können Druckstöße aufgebracht werden.

Der Dialysierflüssigkeitskreislauf 15 enthält eine Dialysierflüssigkeitsquelle 30, eine Pumpe 31 und ein Ventil 32 an der Eingangsseite der Dialysierflüssigkeitskammer 12. Ausgangsseitig von dieser Kammer ist ein zweites Ventil 33 vorgesehen, das in einen Sumpf 34 führt.

Die Drucksensoren 23 und 28 sind mit einer (nicht dargestellten) Steuereinheit verbunden, an die sie die Druckwerte liefern. Die Steuereinheit steuert unter anderem die Pumpe 22 sowie die Ventile 32,33 und 25 nach einem vorbestimmten Programm.

Zur Vorbereitung des Dialysegerätes wird die Anschlussvorrichtung 20 mit dem Spülflüssigkeitsreservoir 27 verbunden und die Anschlussvorrichtung 26 wird mit dem Sumpf 34 verbunden.

Während der Phase des Füllens der Blutseite des Filters 10 sind die Ventile 32 und 33 geschlossen und das Ventil 25 ist geöffnet. Die Pumpe 22 läuft mit einer von der Steuereinheit vorgegebenen Geschwindigkeit, die der ersten Fließrate entspricht. Dadurch wird Spüflüssigkeit von z. B. unten in die Blutkammer 11 gefüllt. Aus der Blutkammer 11 wird die Luft durch die aufsteigende Spülflüssigkeit verdrängt, so dass sie in den Sumpf 27 hinein abgelassen wird. Das Austreiben der Luft beschränkt sich auf die Blutkammer 11. Die Membran 13 ist für die Spülflüssigkeit nahezu undurchlässig oder das Durchtreten der Spülflüssigkeit wird durch einen Druckaufbau auf der Dialysierflüssigkeitsseite verhindert. Der Dialysierflüssigkeitskreislauf benötigt auch keine Entlüftung.

Nach dem vollständigen Befüllen der Blutkammer 11 mit spülflüssigkeit wird die Pumpe 22 auf die zweite Fließrate umgeschaltet, die höher ist als die erste Fließrate. Es erfolgt dann die Spülphase, in der Fremdpartikel aus dem Blutkreislauf 14 herausgespült werden. Die Spülphase kann nach Ablauf einer vorbestimmten Zeit oder einer vorbestimmten Anzahl von Umdrehungen der Pumpe 22 beendet werden.

Bevor der Anschluss eines Patienten an den Blutkreislauf 14 erfolgt, können die Anschlussvorrichtungen 20 und 26 miteinander verbunden werden, so dass sich ein geschlossener Blutkreislauf ergibt, in dem Spülflüssigkeit zirkuliert. Erst beim Anschluss des Patienten an den Blutkreislauf wird die Spülflüssigkeit abgelassen.

Die erste und/oder die zweite Fließrate kann pulsierend verändert werden, indem die Pumpe 22 intermittierend angetrieben wird. Sie kann dabei unterschiedliche Geschwindigkeiten annehmen oder kurzzeitig stillgesetzt werden.

Druckimpulse können durch intermittierendes Öffnen und Schließen des Ventils 25 sowohl während der Füllphase als auch während der Spülphase erzeugt werden.

Die Erfindung verkürzt die zur Vorbereitung eines Dialysegerätes bzw. Filters benötigte Zeit, ohne die Qualität der Vorbereitung zu verringern.

## Patentansprüche

1. Verfahren zum Vorbereiten des Filters (10) eines Dialysegerätes, das einen Blutkreislauf (14) und einen Dialysierflüssigkeitskreislauf (15) aufweist, wobei der Filter (10) eine Blutkammer (11) und eine Dialysierflüssigkeitskammer (12) enthält, zwischen denen sich eine Membran (13) befindet, mit mindestens folgenden Phasen:
a) Füllen der Blutkammer (11) des Filters (10) mit einer Spülflüssigkeit mit einer ersten Fließrate, die so niedrig ist, dass Luftblasen sich ablösen und nach oben entweichen können, und
b) Spülen des Filters durch Hindurchleiten einer Spülflüssigkeit durch die Blutkammer (11) mit einer zweiten Fließrate, die größer ist als die erste Fließrate.

2. Verfahren nach Anspruch 1, **dadurch gekennzeichnet, dass** der Übergang zwischen der Phase des Füllens und der Phase des Spülens zeitgesteuert erfolgt.

3. Verfahren nach Anspruch 1, **dadurch gekennzeichnet, dass** der Übergang zwischen der Phase des Füllens und der Phase des Spülens in Abhängigkeit von der Menge der geförderten spülflüssigkeit erfolgt.

4. Verfahren nach einem der Ansprüche 1 bis 3, **dadurch gekennzeichnet, dass** die erste und/oder zweite Fließrate pulsierend verändert wird.

5. Verfahren nach einem der Ansprüche 1 bis 4, **dadurch gekennzeichnet, dass** durch intermittierendes Öffnen und Schließen mindestens eines Ventils während des Laufens der Pumpe (22) bei fortlaufender Förderung der Spülflüssigkeit Druckimpulse erzeugt werden.

6. Verfahren nach einem der Ansprüche 1 bis 5, **dadurch gekennzeichnet, dass** die erste und die zweite Fließrate von einer volumetrischen Pumpe (22) erzeugt werden.

7. Verfahren nach einem der Ansprüche 1 bis 6, **dadurch gekennzeichnet, dass** während des Spülens eine Umkehr der Fließrichtung erfolgt.

8. Verfahren nach einem der Ansprüche 1 bis 7, **dadurch gekennzeichnet, dass** die zweite Fließrate mindestens doppelt so groß ist wie die erste Fließrate.

9. Verfahren nach einem der Ansprüche 1 bis 8, **dadurch gekennzeichnet, dass** der Übergang zwischen der ersten und zweiten Phase durch einen kontinuierlichen Anstieg der Fließrate erfolgt.

10. Dialysegerät mit einem Filter (10), einem Blutkreislauf (14) und einem Dialysierflüssigkeitskreislauf (15), mindestens einer volumetrischen Pumpe (22), mindestens einem Ventil (25) und mit einer Steuereinheit zur automatischen Steuerung der Pumpe (22) und des mindestens einen Ventils (25), **dadurch gekennzeichnet, dass** die Steuereinheit derart ausgebildet ist, dass sie in einer Füllphase die Pumpe (22) mit einer ersten Fließrate betreibt, während das mindestens eine Ventil (25) geschlossen ist, und in einer Spülphase, in der das mindestens eine Ventil (25) geöffnet ist, die Pumpe (22) mit einer zweiten Fließrate betreibt, die größer ist als die erste Fließrate.
